(19) Europäisches Patentamt
European Patent Office
Office européen des brevets

(11) EP 0 673 355 B1

(12) **FASCICULE DE BREVET EUROPEEN**

(45) Date de publication et mention de la délivrance du brevet:
**12.03.1997 Bulletin 1997/11**

(21) Numéro de dépôt: **93924676.5**

(22) Date de dépôt: **04.11.1993**

(51) Int Cl.6: **C07C 29/36**, C07C 33/02

(86) Numéro de dépôt international:
**PCT/FR93/01090**

(87) Numéro de publication internationale:
**WO 94/18147 (18.08.1994 Gazette 1994/19)**

(54) **PROCEDE DE PREPARATION D'OCTADIENOLS**

VERFAHREN ZUR HERSTELLUNG VON OCTADIENOLEN

METHOD FOR PREPARING OCTADIENOLS

(84) Etats contractants désignés:
**AT BE CH DE DK ES FR GB GR IE IT LI LU NL PT SE**

(30) Priorité: **06.11.1992 FR 9213392**
**04.03.1993 FR 9302524**

(43) Date de publication de la demande:
**27.09.1995 Bulletin 1995/39**

(73) Titulaire: **ELF ATOCHEM S.A.**
**92800 Puteaux (FR)**

(72) Inventeurs:
- **MONFLIER, Eric**
**F-59000 Lille (FR)**
- **BOURDAUDUCQ, Paul**
**F-69630 Chaponost (FR)**
- **COUTURIER, Jean-Luc**
**F-69006 Lyon (FR)**

(74) Mandataire: **Kaplan, Jean-Pierre**
**ELF ATOCHEM S.A.,**
**Département Propriété Industrielle,**
**4, Cours Michelet,**
**La Défense 10**
**F-92091 Paris Cédex 42 (FR)**

(56) Documents cités:
**EP-A- 0 296 550** **EP-A- 0 436 226**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 673 355 B1

## Description

La présente invention concerne un procédé de préparation de n-octadiénols par dimérisation et hydratation du butadiène en présence d'un catalyseur et d'eau.

De nombreux procédés mettant en oeuvre une telle hydrodimérisation et conduisant à un mélange d'octa-2,7-dién-1-ol et d'octa-1,7-dièn-3-ol, ont été décrits dans l'art antérieur.

L'octa-2,7-dièn-1-ol est intéressant, en particulier, comme intermédiaire pour son hydrogénation en n-octan-1-ol, produit utilisé notamment pour la fabrication de plastifiants tels que le di-n-octylphtalate.

On connaît le brevet français 2045369 qui décrit un procédé de préparation de ces octadiènols.

Selon ce procédé mis en pratique, on forme un mélange réactionnel contenant du 1,3-butadiène, de l'eau, un solvant dans lequel le butadiène et l'eau sont au moins partiellement solubles en présence d'un catalyseur comportant des composés de métaux de transition (palladium ou platine) et une phosphine et d'un co-catalyseur constitué par du dioxyde de carbone. La phosphine est choisie parmi des composés connus, insolubles dans l'eau, tels que les trialkylphosphines, les triarylphosphines, les alkylarylphosphines tertiaires.

Le solvant est choisi parmi les éthers dialkyliques, les éthers cycliques, les éthers alkyliques inférieurs de polyalcools ou de polyoxyalkylèneglycols, les alkyloxy- et les aryloxy- polyalcénoxy-alcanols , les cétones, les amides, les dérivés pyridiniques, les sulfoxydes, les sulfones telles que le sulfolane, les esters, les solvants aromatiques, les hydrocarbures aliphatiques, les oléfines.

Des composés connus pour entrer en réaction avec le métal peuvent être ajoutés au mélange réactionnel. Ces composés peuvent servir comme protection supplémentaire pour empêcher le dépôt de métal au cours de la réaction et au cours du traitement subséquent pour la préparation du catalyseur en vue du recyclage.

Ces composés sont énumérés en une liste de plus de 50 produits azotés comprenant entre autres, la triméthylamine, la triéthylamine, la tri-n-octylamine, la diméthyldodécylamine.

Les essais n° 11, 12 et 17 du tableau VII du brevet ci-dessus, réalisés en présence d'acétone et avec ou sans triéthylamine, les autres facteurs restant constants, montrent que l'ajout de triéthylamine diminue à la fois le rendement en octadiènols qui passe de 75% à 70% puis 42%, et également la sélectivité de la formation de l'octa-2,7-dièn-1-ol par rapport à l'octa-1,7-dièn-3-ol, (89/11, 76/24, 73/27). Par contre, le rendement en octatriène, produit secondaire non souhaité, augmente et passe de 2% à 9% puis 15%.

Aucun essai expérimental utilisant la diméthyldodécylamine n'est décrit, et rien ne suggère que cette amine serait moins défavorable que la triéthylamine.

Le brevet français 2366237 décrit une famille d'arylphosphines tertiaires solubles dans l'eau de formule générale (A) :

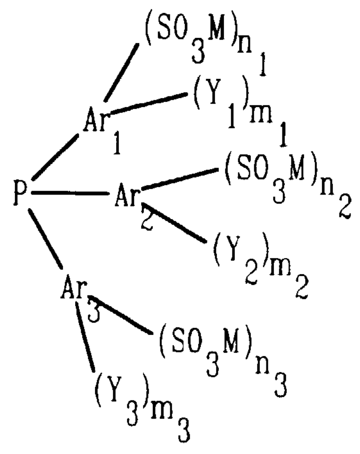

( A )

Cette solubilité est apportée par la présence d'au moins un groupe sulfonate, M étant un reste cationique d'origine minérale ou organique.

Ce brevet divulgue un procédé d'hydrodimérisation du butadiène en présence d'eau et d'un catalyseur constitué par au moins une de ces phosphines solubles et par un métal de transition sous forme métallique ou sous forme d'un composé dudit métal choisi parmi le Pd, le Ni, le Pt, le Co ou le Rh, ledit catalyseur passant en solution dans ladite eau.

Pour accélérer notablement la réaction entre le butadiène et l'eau certains composés solubles dans l'eau sont ajoutés. Ce sont les carbonates et bicarbonates alcalins comme le carbonate et le bicarbonate de sodium, le silicate de sodium et les sels alcalins d'acide phosphoreux, phosphorique et arsénique.

Ce procédé peut être également mis en oeuvre en présence d'amines tertiaires aliphatiques ou aromatiques.

Les exemples 7 à 16, rapportant la synthèse d'octadiènols, sans présence d'amine tertiaire ni de solvant, indiquent au mieux pour l'exemple 10 un taux de conversion de 76% et une sélectivité de 64% en octa-2,7-diènol-1-ol et de 20% pour l'octa-1,7 dièn-3-ol à partir du butadiène consommé, soit une sélectivité ol-1/(ol-1 + ol-3) égale seulement à 76% (100x64/84).

De son côté le brevet FR 2479187 propose un procédé de synthèse des n-octadiènols dans lequel on fait réagir du butadiène et de l'eau dans une solution aqueuse de sulfolane présentant un rapport pondéral eau/sulfolane d'une valeur de 20/80 à 70/30 et contenant des ions carbonate et/ou bicarbonate en présence :

(1°) de palladium ou d'un composé du palladium,
(2°) d'une phosphine monodentée de formule (B)

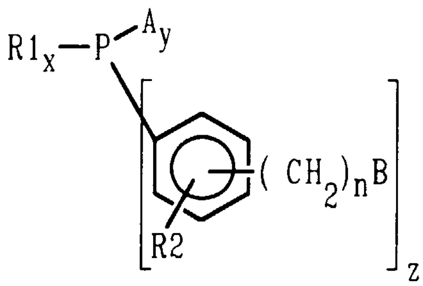

( B )

avec les significations des termes telles qu'indiquées dans ce brevet, et
(3°) d'une amine tertiaire monodentée ayant une constante de basicité (pKa) de 7 ou plus, en une quantité de 1 à 50% en volume basée sur le sulfolane.

Ce brevet indique que le butadiène peut être remplacé par une " fraction-$C_4$" mais que de préférence on met en réaction un butadiène de qualité pour polymérisation ou un butadiène de qualité pour réaction chimique, au vu de la vitesse de réaction et de la facilité de récupération du butadiène n'ayant pas réagi.

Il est précisé que les amines monodentées utiles comprennent les trialkyl (inférieur) amines telles que la triméthylamine, la triéthylamine, la tri-n-propylamine, la tri-n-butylamine. La triéthylamine est préférée sur la base du rendement de la réaction et de ses propriétés intrinsèques telles que point d'ébullition, solubilité et coût.

Les exemples 3 et 7 du tableau 2 de FR 2479187 montrent que le remplacement de la triéthylamine par la tri-n-propylamine provoque une baisse de rendement en octadiènols .

La présence de sulfolane dans ce procédé est un compromis entre les avantages et les inconvénients entraînés par cette présence. En effet, il est indiqué qu'une concentration en sulfolane inférieure à 30% en poids donne une diminution significative de la vitesse de réaction. Cela est illustré par l'exemple 14 de ce même tableau 2 qui indique, qu'en présence d'une concentration en eau de 90% en poids et de triéthylamine, la quantité d'octadiènols obtenue à partir de 25 g de butadiène (462 mmole) n'est que de 1 mmole.

Par contre, une concentration en sulfolane excédant 80% en poids conduit non seulement à une diminution de l'efficacité d'extraction des octadiènols du milieu réactionnel après la fin de la réaction, mais aussi à une augmentation des quantités de palladium et de phosphine dissoutes dans la phase organique d'extraction et à une augmentation des quantités de sous-produits de la réaction.

La demande de brevet EP 0296550 divulgue un procédé de préparation de n-octadiènols semblable au précédent ci-dessus, dans lequel la phosphine monodentée de formule générale (A) est remplacée par un sel de phosphonium de formule générale (C) :

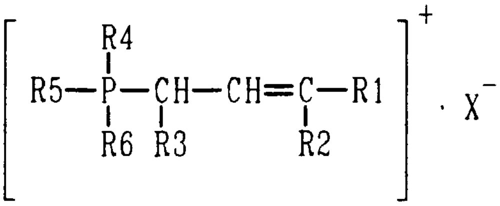

( C )

les substituants R1 à R6 ayant les significations indiquées dans cette demande ci-dessus, X représentant un groupe hydroxyle, hydroxycarbonyloxy ou alkylcarbonyloxy inférieur.

Ce procédé est mis en oeuvre avec un mélange réactionnel comportant un solvant notamment le sulfolane, et comme indiqué aux exemples 7 à 11 et 14, 15, de la triéthylamine et du dioxyde de carbone.

La demande de brevet EP 0411410, traitant de la réaction du 1,3-butadiène avec de l'eau en présence d'un triorganophosphine oxyde, énonce qu'une forme d'exécution très avantageuse économiquement de cette réaction consiste à utiliser des coupes $C_4$ à la place du butadiène pur. Les oléfines 1-butène, 2-butène et isobutène contenues en plus dans la coupe $C_4$ ne participent pas à la réaction ni ne lui portent préjudice.

Les coupes $C_4$ contiennent en poids environ 45% de 1,3-butadiène, 17% de 1-butène, 10% de 2-butène, 25% d'isobutène et comme reste du butane et de l'isobutane.

La demande de brevet EP 0436226 enseigne que les procédés divulgués par les brevets US 4356333 et US 4417079 présentent des inconvénients lors de leur mise en oeuvre en continu à l'échelle industrielle.

En effet, les composants du catalyseur, à savoir, palladium, phosphine, amine tertiaire, et également le solvant (sulfolane), sont élués dans l'extrait obtenu lors de la séparation par extraction des octadiènols du milieu réactionnel. Lorsque cet extrait est soumis à une distillation le palladium métal précipite et finit par encrasser le rebouilleur de distillation.

La solution technique proposée dans cet art antérieur consiste à remplacer la phosphine par un sel de phosphonium de formule générale (C) et à effectuer des lavages de la phase organique d'extraction par une solution aqueuse de sulfolane contenant une phosphine soluble dans l'eau. Ce dernier traitement complique le procédé industriel de synthèse d'octadiènol-1.

Le but de la présente invention est de trouver un procédé industriel de préparation d'octadiènols avec un taux de conversion et/ou un rendement en octadiènols et/ou des sélectivités en octadiènols et octa-2,7-dièn-1-ol voisins ou plus élevés que ceux de l'art antérieur en utilisant un catalyseur particulièrement stable, facile à recycler.

La solution de ce problème réside dans un procédé de préparation d'octa-2,7-dièn-1-ol comportant la réaction d'hydrodimérisation du 1,3-butadiène avec de l'eau en présence d'un métal de transition choisi parmi le palladium, le nickel, le platine, le cobalt et le rhodium, sous forme métallique ou sous forme d'un composé dudit métal, d'un composé phosphoré tertiaire ou quaternaire soluble dans l'eau et au moins d'un composé azoté , caractérisé en ce que le composé azoté est choisi parmi une amine tertiaire et un sel d'ammonium quaternaire de formule générale (I) ou (II) :

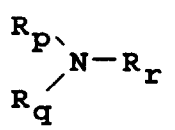

(I)

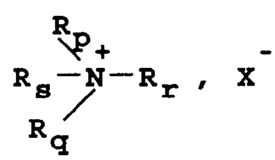

(II)

dans lesquelles $R_p$, $R_q$, et le cas échéant $R_s$, sont identiques ou différents et représentent chacun un groupe méthyle ou éthyle, le groupe $R_r$ représentant un alkyle comprenant de 6 à 22 atomes de carbone, le groupe $X^-$ représentant un contre-anion choisi parmi $HCO_3^-$, $CO_3^{2-}$, $HSO_3^-$, $SO_3^{2-}$, $SiO_3^{2-}$,
$PO_4^{3-}$, $HPO_3^{2-}$, $AsO_4^{3-}$, $SO_4^{2-}$, $HSO_4^-$, $RSO_3^-$, $RCO_2^-$, $OH^-$,
R étant un groupe alkyle, et en ce que l'on opère ladite réaction en présence de dioxyde de carbone.

De manière préférée, le contre-anion est choisi parmi $HCO_3^-$, $CO_3^{2-}$, $RCO_2^-$, $OH^-$.

Le 1,3-butadiène mis en réaction peut être quasiment pur (98% ou plus en poids ) ou faire partie d'un mélange constitué par une coupe $C_4$ ou une coupe $C_4H$ engagée telle que dans ladite réaction d'hydrodimérisation. La coupe $C_4H$ résulte de l'élimination de la coupe $C_4$ des composés acétyléniques par leur l'hydrogénation sélective.

Dans le cadre de la présente invention, il a été trouvé que la coupe $C_4$ ou $C_4H$ conduisait chacune à de meilleurs rendements et sélectivités en octadiènols que le 1,3-butadiène quasiment pur mis en réaction sans la présence d'un solvant auxiliaire.

Le métal de transition est choisi parmi le palladium, le nickel, le platine, le cobalt et le rhodium. Il peut être à différents états d'oxydation. De préférence le métal de transition est le palladium.

De même le composé du métal de transition est choisi parmi les composés des métaux comprenant le palladium,

le nickel, le platine, le cobalt et le rhodium, solubles dans l'eau ou capables de passer en solution dans les conditions de la réaction, comme cela est rapporté dans le brevet FR 2366237, avec ou sans réducteur tel que $NaBH_4$, poudre de Zn, magnésium, $KBH_4$, hydrazine. De préférence le composé du métal de transition est un composé du palladium.

Le choix particulier d'une amine tertiaire de formule (I) ou d'un sel d'ammonium quaternaire de formule (II) permet d'éviter l'ajout dans le milieu réactionnel d'un solvant au moins partiellement miscible à l'eau , notamment de sulfolane.

Selon la présente invention, on peut néanmoins ajouter dans le milieu réactionnel un tel solvant hydrophile, notamment un solvant polaire aprotique tel que le diméthylformamide (DMF), le diméthylsulfoxyde, l'acétonitrile, le sulfolane, ou choisi parmi les polyéthylèneglycoléthers, par exemple le tétraglyme.

Cependant, de manière avantageuse, la réaction du butadiène et de l'eau est pratiquée sans solvant miscible ou partiellement miscible à l'eau.

En effet, cette absence de solvant hydrophile pendant la réaction permet de simplifier le traitement post-réactionnel conduisant à l'isolement des octadiènols.

Par contre, il a été trouvé que la réaction d'hydrodimérisation du butadiène quasiment pur était favorisée par la présence d'un hydrocarbure monoéthylénique ou saturé ayant de 4 à 7 atomes de carbones. Cet hydrocarbure, immiscible à l'eau, favorise de plus le traitement ultérieur du mélange réactionnel après la fin de la réaction d'hydrodimérisation. Cet hydrocarbure peut avoir un squelette carboné linéaire, ramifié ou cyclique. Il peut être notamment du butane, du pentane, du cyclopentane, de l'hexane, du cyclohexane, de l'heptane ou leurs homologues monoéthyléniques.

Le composé phosphoré tertiaire ou quaternaire peut être une phosphine ou un sel de phosphonium de formule générale (A), (B) ou (C) ci-dessus et tel que décrit dans l'art antérieur.

Ce composé peut être ainsi une triarylphosphine substituée par au moins un groupe sulfonate de métal alcalin tel que sodium, potassium ou lithium. Le contre-anion du groupe sulfonate peut être également choisi parmi les groupes ammonium quaternaire.

Les amines de formule générale (I) et les ammoniums quaternaires de formule (II) ont des groupes $R_p$, $R_q$ et le cas echeant $R_s$, qui représentent chacun indépendamment l'un de l'autre un groupe méthyle ou éthyle.

De préférence, $R_p$, $R_q$ et le cas échéant $R_s$ représentent chacun un groupe méthyle.

L'amine (I) peut être solubilisée dans l'eau par salification à l'aide d'un acide notamment anhydride carbonique ($CO_2$).

Certaines de ces amines (I) sont disponibles commercialement, soit pures soit en mélanges.

D'une manière générale, les amines (I) peuvent être synthétisées par alkylation d'une diamine de formule $R_pR_qNH$ à l'aide d'un agent alkylant de formule $R_rX$, X étant un groupe partant par exemple halogène.

Les amines (I) peuvent être également obtenues à partir de l'amine primaire $R_r$-$NH_2$ par réaction avec du formaldéhyde ou de l'acétaldéhyde et hydrogénation catalytique.

Certains de ces sels d'ammonium quaternaires (II) sont disponibles commercialement. Si ces sels ont comme contre-anion un halogénure, notamment $Cl^-$, $Br^-$, $I^-$, $F^-$, ce dernier est échangé au préalable, par exemple, à l'aide d' une résine échangeuse d'ions, en un autre contre-anion appartenant à la famille citée ci-dessus, afin de ne pas inhiber la réaction d'hydrodimérisation.

D'une manière générale les sels (II) peuvent être préparés d'une manière classique, par alkylation de l'amine tertiaire (I) par un agent alkylant de formule R-Z, Z étant un groupe partant notamment halogénure ou sulfate. Les agents alkylants peuvent être par exemple le diméthylsulfate ou le diéthylsulfate.

La réaction selon l'invention peut être réalisée à l'aide d'une amine ou d'un mélange d'amines (I) ou encore d'un sel d'ammonium (II) ou d'un mélange de sels(II).

De préférence le groupe alkyle $R_r$ représente le radical $-(CH_2)_nCH_3$ dans lequel n vaut un nombre entier compris entre 5 et 21.

Avantageusement n est un nombre entier compris entre 9 et 17 notamment 11.

De préférence, la réaction est conduite à une température comprise entre 20 et 100°C.

Après la fin de la réaction, le milieu réactionnel est mis, si nécessaire, à la pression et à la température ambiantes. Le milieu réactionnel est soumis directement à une distillation éclair (flash). La phase organique recueillie est ensuite rectifiée par distillation. Le résidu de la première distillation éclair comportant essentiellement le composé phosphoré, le métal de transition et l'amine tertiaire (I) ou le sel d'ammonium (II), peut être recyclé dans une nouvelle réaction avec du butadiène et de l'eau.

En plus de la description qui précède les exemples suivants, donnés à titre purement illustratif, permettent de mieux comprendre la présente invention. Dans ces exemples, les produits ont été identifiés par RMN $^{13}C$ à l'aide d'un appareil AC 300 BRUKER, par spectre IR sur un NICOLET 20 SXB , par spectrographie de masse à l'aide d'un FISON VG 12250 et par chromatographie en phase gazeuse sur colonne de silicone, avec comme étalon interne du n-octanol. L'autoclave utilisé est le modèle "Autoclave Engineer" de 100 ml ou de 300 ml. L'acétate de palladium (II) ( Aldrich ) est à 98% de pureté.

Le 1,3-Butadiène ( Union Carbide ) a une pureté supérieure à 99%.

Parmi les abréviations utilisées :

Le TPPTS est le sel trisodique de la tri(métasulfophényl)phosphine. Ce sel est préparé à titre expérimental selon le brevet FR 2366237.

Le TPPMS est le sel monosodique de la métasulfophényldiphényl phosphine. Ce sel est préparé selon ARHLAND et coll., Journal of the Chemical Society, 276-288, (1958).

NORAM DMCD représente la diméthylcocoamine, coco étant un mélange de groupes alkyle linéaire saturé en $C_8$ (4%), $C_{10}$ (6%), $C_{12}$ (55%), $C_{14}$ (18%) et $C_{16}$ (10%), $C_{18}$ (7%).

NORAM DMSHD représente la diméthylsuifhydrogéné amine (suifhydrogéné signifie un mélange de groupes alkyle linéaire saturé en $C_{12}$ (0,1%),$C_{14}$ (0,9%), $C_{16}$ (28%) et de $C_{18}$ (71%).

NORAM $MC_2$ représente la dicocométhylamine.

Ces produits NORAM sont disponibles commercialement auprès de la Société CECA/ELF ATOCHEM (FRANCE).

Les coupes $C_4$ et $C_4H$ ont les compositions pondérales suivantes, indiquées dans le tableau I ci-après:

TABLEAU I

| | $C_4$ | $C_4H$ |
|---|---|---|
| Cyclopropane | 0,04 | 0,02 |
| Propylène | - | 0,04 |
| Isobutane | 0,57 | 0,91 |
| Propadiène | 0,02 | 0,01 |
| n-Butane | 3,02 | 4,61 |
| Non-identifié | 0,03 | 0,03 |
| trans 2- Butène | 5,06 | 6,55 |
| 1- Butène | 12,32 | 15,13 |
| Isobutène | 26,98 | 27,15 |
| cis 2- Butène | 4,08 | 4,47 |
| 1,2- Butadiène | 0,30 | 0,14 |
| Propyne | 0,02 | - |
| 1,3- Butadiène | 46,71 | 40,91 |
| Vinylacétylène | 0,69 | - |
| Ethylacétylène | 0,16 | - |
| Total | 100,0 | 99,97 |

Dans ce qui suit:

La durée totale de réaction est égale à la durée de chauffage de l'autoclave.

La température en °C est celle de l'autoclave, mesurée à l'aide d'un thermocouple étalonné.

Le taux de conversion, en %, est égal au rapport multiplié par 100 du nombre de moles de butadiène consommé sur le nombre de moles du butadiène introduit dans l'autoclave.

Le rendement en octadiènols, en %, est égal au rapport multiplié par 100 du nombre de moles de butadiène transformé en octadiènols sur le nombre de moles du butadiène introduit.

La sélectivité en octadiènols, en %, est égale au rapport multiplié par 100 du nombre de moles de butadiène transformé en octadiènols sur le nombre de moles de butadiène consommé.

La sélectivité en ol-1/ols, en %, est égale au rapport multiplié par 100 du nombre de moles d'octadiènol-1 sur le nombre de moles d'octadiènols-1 et-3.

Le rendement en dimères, en %, est égal au rapport multiplié par 100 du nombre de moles de butadiène transformé en dimères sur le nombre de moles du butadiène introduit.

Les dimères sont constitués essentiellement par du vinylcyclohéxène et des octatriènes.

Les éthers dioctadiènyliques résultent de la déshydratation des octadiènols.

Le rendement en éthers, en %, est égal au rapport multiplié par 100 du nombre de moles de butadiène transformé en éthers sur le nombre de moles du butadiène introduit.

L'expression "divers" représente des produits non identifiés.

Exemple 1

On introduit, sous atmosphère d'argon, dans un autoclave de 100 ml en acier inoxydable, équipé d'une agitation mécanique, 0,146 g ($6,5.10^{-4}$ mol) d'acétate de palladium ($Pd(OAc)_2$), 1,25 g ($2.10^{-3}$ mol) de TPPTS, 6,5 g (0,03 mol) de diméthyldodécylamine, 12 g (0,666 mol) de $H_2O$ et 18 g (0,333 mol) de butadiène.

L'autoclave est alors pressurisé sous 10 bars de $CO_2$.

Tout en agitant le mélange réactionnel à 800 tours/minute, la température de l'autoclave est élevée à 85°C progressivement sur une durée de 15 min. On poursuit la réaction à 85°C pendant 30 min.

La réaction est suivie en surveillant la diminution de la pression à l'intérieur de l'autoclave.

Lorsque la pression n'évolue plus, on refroidit l'autoclave à température ambiante, puis on le dégaze par ouverture de la vanne. Le brut réactionnel, recueilli sous atmosphère de $CO_2$, comporte deux phases : une phase organique surnageante au-dessus d'une phase aqueuse. La phase organique analysée par chromatographie indique un rendement de 72% d'octadiènols (67% d'octa-2,7-dièn-1-ol, 5% d'octa-1,7-dièn-3-ol), 17% d'un mélange d'octatriène et de vinylcyclohéxène et 3% d'éthers dioctadiènyliques. La sélectivité en ol-1/ols est de 93%.

Le tableau II suivant reprend les conditions expérimentales de l'exemple 1 et montre huit autres exemples de réalisation sans solvant du procédé selon l'invention. Les quantités molaires de réactifs sont les mêmes que celles de l'exemple 1 sauf si précisées autrement.

Dans l'exemple 3, une partie du brut réactionnel a été stockée 1 mois à la température ambiante, sous atmosphère de $CO_2$. Il ne présente alors aucun précipité noir, signe de décomposition du catalyseur.

Le tableau III montre les exemples 10 et 12 selon la présente invention et l'exemple comparatif 11, réalisés en présence de solvant (sulfolane). Les quantités de sulfolane et les quantités molaires de réactifs utilisés sont celles du premier exemple du brevet FR 2479187 multipliées par 1,5. On utilise un autoclave de 300 ml du type indiqué ci-dessus. A titre comparatif, l'exemple 11 a été reproduit trois fois (moyenne des trois essais réalisés). Tous les trois bruts réactionnels de cet exemple 11 contiennent déjà, lorsqu'on les sort de l'autoclave, un précipité noir, signe de décomposition du catalyseur.

Les résultats de l'exemple 10 sont meilleurs que ceux de l'exemple 11.

Le tableau IV montre 10 exemples comparatifs (n° 13 à 21) pratiqués sans solvant et avec les mêmes quantités molaires de réactifs que l'exemple 1 du tableau I, à l'exception des quantités d'amine ou de carbonate de potassium, égales à 0,01 mole.

Pour une même quantité molaire d'amine, les exemples 4 à 9 conduisent à un taux de conversion et/ou à un rendement en octadiènols et/ou à des sélectivités plus élevés que les exemples comparatifs 15 à 21.

Exemple 22

On introduit, sous atmosphère d'argon, dans un autoclave de 300 ml en acier inoxydable, équipé d'une agitation mécanique, 0,30 g ( 1,35 mmoles ) d'acétate de palladium ( $Pd(OAc)_2$ ) , 2,4 g ( 4,2 mmoles) de TPPTS , 23,4 g ( 105 mmoles ) de diméthyldodécylamine, 25,2 g ( 1,4 moles ) de $H_2O$ et 80,9 g de coupe $C_4$ contenant 37,8 g (700 mmoles) de butadiène. L'autoclave est alors pressurisé sous 10 bars de $CO_2$.

Tout en agitant le mélange réactionnel à 800 tours / minute, la température de l'autoclave est élevée à 70°C progressivement sur une durée de 30 minutes.

On poursuit la réaction à 70°C pendant 150 minutes.

On refroidit l'autoclave à température ambiante, puis on le dégaze.

Le brut réactionnel, recueilli sous atmosphère de $CO_2$ , comporte deux phases: une phase organique surnageante au-dessus d'une phase aqueuse.

La phase organique est analysée par chromatographie et les résultats apparaissent dans le tableau V ci-après.

Ce tableau montre, en plus de l'exemple 22 , neuf autres exemples 23 à 31.

Les conditions opératoires sont les mêmes que celles de l'exemple 22 et en particulier les quantités molaires de réactifs sont identiques à celles de l'exemple 22, sauf si précisées autrement.

Dans les exemples 22,23,25 à 28,30 et 31, les mélanges $C_4$, $C_4H$, butadiène et pentane, butadiène et 1-pentène, comportent chacun une même quantité de 1,3 - butadiène ( 700 mmoles ) que les exemples 24,29, réalisés avec du butadiène pur à 99%.

Ces mélanges donnent de meilleurs résultats en termes de taux de conversion, rendements en octadiènols et sélectivité en octadiènols que le 1,3-butadiène pur.

Exemple 32

Préparation de l'Hydroxyde de cétyltriméthylammonium.

Le bromure de cétyltriméthylammonium ( Aldrich ) est mis en solution dans l'eau à une concentration d' environ 0,2 mol/litre, puis cette solution est passée sur une colonne échangeuse d'ions Amberlite IRA 420 (OH).

La solution recueillie est concentrée sous vide jusqu'à formation d'un gel.

L' analyse montre que les ions $Br^-$ ont été totalement échangés et remplacés par des ions $OH^-$, pour donner une solution d' hydroxyde de cétyltriméthylammonium à 18% en poids par rapport au poids de la solution.

Exemple 33

On introduit, sous atmosphère d'argon, dans un autoclave de 300 ml en acier inoxydable, équipé d'une agitation mécanique, 0,44g ($1{,}96.10^{-3}$ mol) d'acétate de palladium ($Pd(OAc)_2$), 2,13 g ($5{,}85.10^{-3}$ mol)de TPPMS, 81 g(4,5 mol) d'eau, 54 g (1 mol) de 1,3-butadiène et 18,1 g (0,06 mol) d'hydroxyde de cétyltriméthylammonium (formule (II): $R_r$ = $-n-C_{16}H_{33}$ , $R_p = R_q = R_s = -CH_3$, $X^- = OH^-$).

L' autoclave est alors préssurisé sous 10 bars de dioxyde de carbone. Tout en agitant le mélange réactionnel à 800 tours/minute, la température de l'autoclave est élevée à 70°C et la réaction est alors laissée 90 minutes à cette température. Aprés retour à la température ambiante, 1' autoclave est dégazé. Le brut réactionnel recueilli comporte deux phases: une phase organique surnageante et une phase aqueuse. La phase organique est analysée par chromatographie en phase gaz comme indiquée ci-dessus.

Exemple comparatif 34

Cet exemple est pratiqué de manière identique à l'exemple 33, sauf que 1' hydroxyde de cétyltriméthylammonium est remplacé par une même quantité molaire d' hydroxyde de tétraméthylammonium.

Les résultats obtenus figurent dans le tableau VI ci-après.

Tableau II

| Sans solvant | | | | | | | | | | |
|---|---|---|---|---|---|---|---|---|---|---|
| Exemple N° | Additifs | Phosphine | Durée totale de réaction en minutes | Température en °C | Taux de conversion en % | Rendement en octadiènols en % | Sélectivité en octadiènols en % | Sélectivité en ol-1/ols en % | Rendement en dimères en % | Rendement en éthers dioctadiénylique en % |
| 1 | $CO_2$ Diméthyldodécyl-amine | TPPTS | 45 | 85 | 92 | 72 | 79 | 93 | 17 | 3 |
| 2 | $CO_2$ Diméthyldodécyl-amine | TPPTS(b) | 240 | 55 | 95 | 76 | 80 | 96 | 17 | 2 |
| 3 | $CO_2$ Diméthyldodécyl-amine(c) | TPPTS | 45 | 70 | 85 | 69 | 81 | 93 | 16 | traces |
| 4 | $CO_2$ Diméthyldodécyl-amine(d) | TPPTS | 90 | 85 | 89 | 63 | 71 | 94 | 16 | 8 |
| 5 | $CO_2$ Diméthylhéxyl-amine(d) | TPPMS | 90 | 85 | 66 | 56 | 85 | 91 | 7 | 3 |
| 6 | $CO_2$ Diméthyloctyl-amine(d) | TPPMS | 90 | 85 | 65 | 54 | 83 | 90 | 5 | 6 |
| 7 | $CO_2$ NORAM DMCD(d) | TPPTS | 90 | 85 | 85 | 59 | 70 | 94 | 15 | 8 |
| 8 | $CO_2$ NORAM DMCD(d) | TPPMS | 90 | 85 | 81 | 65 | 80 | 91 | 10 | 6 |
| 9 | $CO_2$ NORAM DMSHD(d) | TPPTS | 90 | 85 | 86 | 62 | 72 | 94 | 15 | 8 |

(b) $1,33 . 10^{-3}$ mol de TPPTS (c) 0,05 mol d'amine (d) 0,01 mol d'amine

Tableau III

| En présence de Sulfolane | | | | | | | | | | |
|---|---|---|---|---|---|---|---|---|---|---|
| Exemple N° | Additifs | Phosphine | Durée totale de réaction en minutes | Température en °C | Taux de conversion en % | Rendement en octadiènols en % | Sélectivité en octadiènols en % | Sélectivité en ol-1/ols en % | Rendement en dimères en % | Rendement en éthers dioctadiénylique en % |
| 10 | $CO_2$ Diméthyldodécylamine | TPPMS | 195 | 85 | 61 | 47 | 77 | 95 | 14 | 0,4 |
| 11* | $CO_2$ $N(Et)_3$ | TPPMS | 195 | 85 | 44 | 33 | 76 | 94 | 10 | 0,2 |
| 12 | $CO_2$ Diméthyldodécylamine | TPPTS | 195 | 85 | 66 | 49 | 74 | 95 | 15 | 0,4 |

* Exemple comparatif

Tableau IV

| Sans solvant | | | | | | | | | | |
|---|---|---|---|---|---|---|---|---|---|---|
| Exemple N° | Additifs | Phosphine | Durée totale de réaction en minutes | Température en °C | Taux de conversion en % | Rendement en octadiènols en % | Sélectivité en octadiènols en % | Sélectivité en ol-1/ols en % | Rendement en dimères en % | Rendement en éthers dioctadié-nylique en % |
| 13* | $K_2CO_3$ | TPPTS | 195 | 85 | 26 | 20 | 77 | 70 | 6 | - |
| 14* | $CO_2$ | TPPTS | 195 | 85 | 6 | traces | - | - | 5 | - |
| 15* | $CO_2$ $N(Et)_3$ | TPPTS | 195 | 85 | 11 | 5 | 50 | 82 | 5 | - |
| 16* | $CO_2$ $N(n\text{-}Bu)_3$ | TPPTS | 195 | 85 | 7 | 1 | 14 | 46 | 6 | - |
| 17* | $CO_2$ $N(Et)_3$ | TPPMS | 90 | 85 | 33 | 20 | 60 | 85 | 10 | 3 |
| 18* | $CO_2$ Diméthylbutyl-amine | TPPMS | 90 | 85 | 28 | 22 | 79 | 91 | 5 | 1 |
| 19* | $CO_2$ $N(n\text{-}Bu)_3$ | TPPMS | 90 | 85 | 22 | 12 | 54 | 25 | 10 | - |
| 20* | $CO_2$ $N(n\text{-}Octyl)_3$ | TPPMS | 90 | 85 | 17 | 1 | 6 | - | 10 | - |
| 21* | $CO_2$ NORAM $MC_2$ | TPPTS | 195 | 85 | 4 | - | - | - | 4 | - |

* Exemples comparatifs. Additifs: $K_2CO_3$ ou Amine = 0,01 mole

TABLEAU V

| Exemple N° | Phosphine | 1,3 - Butadiène engagé sous la forme de : | Taux de conversion en % | Rendement en octadiénols en % | Sélectivité en octadiénols en % | Sélectivité en ol-1/ols en % | Rendement en dimères en % | Rendement en éthers en % | Rendement en divers en % |
|---|---|---|---|---|---|---|---|---|---|
| 22 | TPPTS | $C_4$ | 87,8 | 70,6 | 80,4 | 92,4 | 9,3 | 3,1 | 4,8 |
| 23 | TPPTS | $C_4H$ | 89,0 | 73,7 | 82,8 | 91,7 | 7,9 | 3,4 | 4,0 |
| 24 | TPPTS | 1,3 - butadiène | 74,6 | 55,4 | 74,3 | 92,8 | 15,1 | 2,1 | 2,0 |
| 25 | TPPTS | 1,3 - butadiène + pentane (a) | 87,9 | 70,3 | 80,0 | 91,5 | 8,9 | 3,2 | 5,5 |
| 26 | TPPMS | $C_4$ | 95,6 | 73,6 | 77,0 | 86,4 | 11,8 | 3,5 | 6,7 |
| 27 | TPPMS | $C_4H$ | 86,4 | 67,5 | 78,1 | 82,7 | 9,3 | 3,5 | 6,1 |
| 28 | TPPMS | $C_4H$ (b) | 71,7 | 57,8 | 80,6 | 86,7 | 4,8 | 3,6 | 5,5 |
| 29 | TPPMS | 1,3 - butadiène | 81,4 | 54,6 | 67,1 | 79,1 | 15,4 | 3,4 | 8,0 |
| 30 | TPPMS | 1,3 - butadiène + pentane (c) | 94,4 | 68,3 | 72,3 | 80,5 | 14,3 | 5,0 | 6,8 |
| 31 | TPPMS | 1,3 - butadiène +1-pentène (d) | 92,5 | 66,5 | 71,9 | 79,1 | 15,2 | 4,7 | 6,1 |

(a) pentane = 54,5 g

(b) 0,042 mole d'amine $C_{12}H_{25}N(CH_3)_2$

(c) pentane = 60,4 g

(d) 1-pentène = 54,1 g

TABLEAU VI

| Exemple N° | Phosphine | 1,3 - Butadiène engagé sous la forme de : | Taux de conversion en % | Rendement en octadiénols en % | Sélectivité en octadiénols en % | Sélectivité en ol-1/ols en % | Rendement en dimères en % | Rendement en éthers en % | Rendement en divers en % |
|---|---|---|---|---|---|---|---|---|---|
| 33 | TPPMS | 1,3 -butadiène | 84 | 60 | 71 | 90 | 7 | 13 | 4 |
| 34* | TPPMS | 1,3 -butadiène | 16 | 9 | 56 | 89 | 4 | 2 | 1 |

* exemple comparatif

## Revendications

1. Procédé de préparation d'octa-2,7-dièn-1-ol comportant la réaction d'hydrodimérisation du 1,3-butadiène avec de l'eau en présence d'un métal de transition choisi parmi le palladium, le nickel, le platine, le cobalt et le rhodium, sous forme métallique ou sous forme d'un composé dudit métal, d'un composé phosphoré tertiaire ou quaternaire soluble dans l'eau et au moins d'un composé azoté , caractérisé en ce que le composé azoté est choisi parmi une amine tertiaire et un sel d'ammonium quaternaire de formule générale (I) ou (II) :

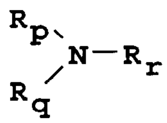

(I)

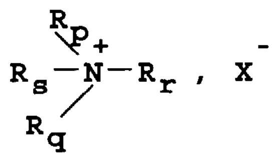

(II)

dans lesquelles $R_p$, $R_q$, et le cas échéant $R_s$, sont identiques ou différents et représentent chacun un groupe méthyle ou éthyle, le groupe $R_r$ représentant un alkyle comprenant de 6 à 22 atomes de carbone, le groupe $X^-$ représentant un contre-anion choisi parmi $HCO_3^-$, $CO_3^{2-}$, $HSO_3^-$, $SO_3^{2-}$, $SiO_3^{2-}$,
$PO_4^{3-}$, $HPO_3^{2-}$, $AsO_4^{3-}$, $SO_4^{2-}$, $HSO_4^-$, $RSO_3^-$, $RCO_2^-$, $OH^-$,
R étant un groupe alkyle, et en ce que l'on opère ladite réaction en présence de dioxyde de carbone.

2. Procédé suivant la revendication 1 caractérisé en ce que le 1,3-butadiène à une pureté égale ou supérieure à 98% en poids.

3. Procédé suivant la revendication 1, caractérisé en ce que le 1,3-butadiène fait partie d'une coupe $C_4$ ou $C_4H$ engagée telle que dans ladite réaction d'hydrodimérisation.

4. Procédé suivant l'une des revendications 1 à 3, caractérisé en ce que ladite réaction du butadiène et de l'eau est pratiquée sans l'ajout d'un solvant miscible ou partiellement miscible dans l'eau.

5. Procédé suivant la revendication 2, caractérisé en ce que ladite réaction d'hydrodimérisation est pratiquée en présence d'un hydrocarbure monoéthylénique ou saturé ayant de 4 à 7 atomes de carbones.

6. Procédé suivant l'une des revendications 1 à 5, caractérisé en ce que le métal de transition est le palladium.

7. Procédé suivant l'une des revendications 1 à 6, caractérisé en ce que le composé du métal de transition est un composé du palladium.

8. Procédé suivant l'une des revendications 1 à 7, caractérisé en ce que $R_p$, $R_q$ et le cas échéant $R_s$ représentent chacun un groupe méthyle.

9. Procédé suivant l'une des revendications 1 à 8, caractérisé en ce que le groupe $R_r$ représente le radical $-(CH_2)_nCH_3$ dans lequel n vaut un nombre entier compris entre 5 et 21.

10. Procédé suivant la revendication 9, caractérisé en ce que n est compris entre 9 et 17.

11. Procédé suivant la revendication 10, caractérisé en ce que n vaut 11.

12. Procédé suivant l'une des revendications 1 à 11, caractérisé en ce que ladite réaction est pratiquée à une température comprise entre 20 et 100°C.

## Patentansprüche

1. Verfahren zur Herstellung von Octa-2,7-dien-1-ol durch Hydrodimerisation von 1,3-Butadien mit Wasser in Gegenwart eines Übergangsmetalls, das unter Palladium, Nikkel, Platin, Cobalt und Rhodium ausgewählt ist und das in metallischer Form oder in Form einer Verbindung des Metalls vorliegt, einer in Wasser löslichen tertiären oder quaternären Phosphorverbindung und mindestens einer Stickstoffverbindung,
   dadurch gekennzeichnet, daß
   die Stickstoffverbindung unter tertiären Aminen und quaternären Ammoniumsalzen der allgemeinen Formeln (I) bzw. (II)

   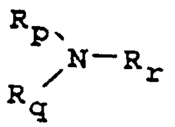

   (I)

   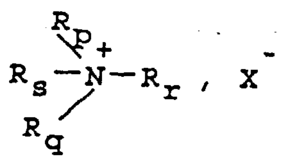

   (II)

   ausgewählt ist, worin $R_p$, $R_q$ und gegebenenfalls $R_s$, die gleich oder voneinander verschieden'sind, jeweils eine Methyl- oder Ethylgruppe und $R_r$ eine Alkylgruppe mit 6 bis 22 Kohlenstoffatomen bedeuten und das Gegenion $X^-$ unter
   $HCO_3^-$, $CO_3^{2-}$, $HSO_3^-$, $SO_3^{2-}$, $SiO_3^{2-}$,

   $PO_4^{3-}$, $HPO_3^{2-}$, $AsO_4^{3-}$, $SO_4^{2-}$, $HSO_4^-$, $RSO_3^-$, $RCO_2^-$, $OH^-$,

   ausgewählt ist, wobei R eine Alkylgruppe bedeutet, sowie dadurch, daß
   die Reaktion in Gegenwart von Kohlendioxid durchgeführt wird.

2. Verfahren nach Anspruch 1,
   dadurch gekennzeichnet, daß das 1,3-Butadien eine Reinheit von 98 Gew.-% oder darüber aufweist.

3. Verfahren nach Anspruch 1, dadurch gekennzeichnet, daß das 1,3-Butadien in einem $C_4$- oder $C_4$H-Schnitt enthalten ist, der bei der Hydrodimerisation eingesetzt wird.

4. Verfahren nach einem der Ansprüche 1 bis 3, dadurch gekennzeichnet, daß die Umsetzung des Butadiens mit dem Wasser ohne Zusatz eines teilweise oder vollständig mit Wasser mischbaren Lösungsmittels durchgeführt wird.

5. Verfahren nach Anspruch 2, dadurch gekennzeichnet, daß die Hydrodimerisation in Gegenwart eines einfach ethylenisch ungesättigten oder eines gesättigten Kohlenwasserstoffs mit 4 bis 7 Kohlenstoffatomen durchgeführt wird.

6. Verfahren nach einem der Ansprüche 1 bis 5, dadurch gekennzeichnet, daß das Übergangsmetall Palladium ist.

7. Verfahren nach einem der Ansprüche 1 bis 6, dadurch gekennzeichnet, daß die Übergangsmetallverbindung eine Palladiumverbindung ist.

8. Verfahren nach einem der Ansprüche 1 bis 7, dadurch gekennzeichnet, daß $R_p$, $R_q$ und gegebenenfalls $R_s$ jeweils eine Methylgruppe bedeuten.

9. Verfahren nach einem der Ansprüche 1 bis 8, dadurch gekennzeichnet, daß $R_r$ die Gruppe $-(CH_2)_nCH_3$ bedeutet, worin n eine ganze Zahl im Bereich von 5 bis 21 darstellt.

10. Verfahren nach Anspruch 9, dadurch gekennzeichnet, daß n im Bereich von 9 bis 17 liegt.

**11.** Verfahren nach Anspruch 10, dadurch gekennzeichnet, daß n gleich 11 ist.

**12.** Verfahren nach einem der Ansprüche 1 bis 11, dadurch gekennzeichnet, daß die Reaktion bei einer Temperatur im Bereich von 20 bis 100 °C durchgeführt wird.

**Claims**

**1.** Process for the preparation of octa-2,7-dien-1-ol comprising the hydrodimerization reaction of 1,3-butadiene with water in the presence of a transition metal, chosen from palladium, nickel, platinum, cobalt and rhodium, in the metal form or in the form of a compound of the said metal, of a water-soluble tertiary or quaternary phosphorus-containing compound and of at least one nitrogen-containing compound, characterized in that the nitrogen-containing compound is chosen from a tertiary amine and a quaternary ammonium salt of general formula (I) or (II):

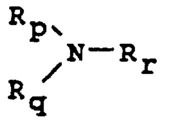

(I)

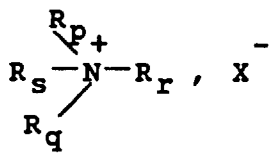

(II)

in which $R_p$, $R_q$ and , if appropriate, $R_s$ are identical or different and each represent a methyl or ethyl group, the group $R_r$ representing an alkyl comprising from 6 to 22 carbon atoms, the group $X^-$ representing a counter-anion chosen from
$HCO_3^-$, $CO_3^{2-}$, $HSO_3^-$, $SO_3^{2-}$, $SiO_3^{2-}$,
$PO_4^{3-}$, $HPO_3^{2-}$, $AsO_4^{3-}$, $SO_4^{2-}$, $HSO_4^-$, $RSO_3^-$, $RCO_2^-$, $OH^-$,
R being an alkyl group, and in that the said reaction is carried out in the presence of carbon dioxide.

**2.** Process according to Claim 1, characterized in that the 1,3-butadiene has a purity equal to or greater than 98 % by weight.

**3.** Process according to Claim 1, characterized in that the 1,3-butadiene forms part of a $C_4$ or $C_4H$ cut used as such in the said hydrodimerization reaction.

**4.** Process according to one of Claims 1 to 3, characterized in that the said reaction of butadiene and water is carried out without the addition of a water-miscible or partially water-miscible solvent.

**5.** Process according to Claim 2, characterized in that the said hydrodimerization reaction is carried out in the presence of a monoethylenic or saturated hydrocarbon having from 4 to 7 carbon atoms.

**6.** Process according to one of Claims 1 to 5, characterized in that the transition metal is palladium.

**7.** Process according to one of Claims 1 to 6, characterized in that the transition metal compound is a palladium compound.

**8.** Process according to one of Claims 1 to 7, characterized in that $R_p$, $R_q$ and, if appropriate, $R_s$ each represent a methyl group.

**9.** Process according to one of Claims 1 to 8, characterized in that the $R_r$ group represents the radical $-(CH_2)_nCH_3$ in which n has the value of an integer between 5 and 21.

10. Process according to Claim 9, characterized in that n is between 9 and 17.

11. Process according to Claim 10, characterized in that n has the value 11.

12. Process according to one of Claims 1 to 11, characterized in that the said reaction is carried out at a temperature between 20 and 100°C.